# EUROPEAN PATENT SPECIFICATION

(11) **EP 1 358 851 B1**
(45) Date of publication and mention of the grant of the patent: **10.08.2005**
(21) Application number: 02009957.8
(22) Date of filing: 03.05.2002
(51) Int. Cl.: A61B 17/132

(54) **Haemostatic device for an open blood vessel**
Vorrichtung zur Hämostase eines offenen Blutgefässes
Dispositif destiné à la hémostase d'un vaisseau sanguin ouvert

(43) Date of publication of application: 05.11.2003
(73) Proprietor: Lina Medical ApS, 2600 Glostrup (DK)
(72) Inventor: Fleischer, Philip, 2300 Copenhagen S (DK)
(74) Representative: Nielsen, Henrik Sten

(56) References cited:
- WO-A-98/46144
- WO-A-98/52477
- DE-C- 56 336
- DE-C- 219 012
- DE-C- 3 907 522
- US-A- 5 133 734
- US-A- 5 512 056
- US-A- 5 569 297
- US-A- 5 873 890

## Description

The present invention relates to a device for establishing hemostasis of an open artery and/or a vein of an extremity of a person through mechanically pressing an area of the extremity at the open artery and/or vein.

The present invention relates to a refined and novel technique for establishing hemostasis at a puncture site or a wound of a person through mechanically compressing the puncture site or wound. In specific surgical applications, the artery and/or the vein of a patient or person are opened for providing access to the blood circuit and/or for guiding blood from the artery system of the patient or person to an external apparatus, such as a dialysis apparatus and for guiding the blood back to the patient. After the dialysis treatment has been finalised or a similar treatment has been terminated involving the opening of arteries or veins of a patient, in particular at the extremity of a patient or person, such as at the wrist of the person, hemostasis is to be established before the patient or person is allowed to leave the hospital or left alone in a hospital. Conventionally, the establishing of hemostasis is established as a nurse presses his or her thumb to the site or area of the open artery and/or the open vein and maintains the pressure for a period of time of 15-30 minutes for establishing hemostasis. The manual process of pressing a finger, in particular the thumb, against the artery and/or the vein at the wrist of a person is for many reasons an unsatisfactory process, as the manual pressure application involves a waste of great many labour years in a hospital or a clinic and further, from a technical point of view, is far from constituting an optimum pressure application, as the nurse or other individual applying the manual pressure to the wrist of the patient or person, is unable to apply the correct pressure for an extended period of time. The risk of blood vessel occlusion and discomfort for the patient are therefore pronounced

A number of compressive appliances or hemostasis establishing devices have been developed for the use in the treatment of dialysis patients for establishing hemostatis of the open artery and the open vein used for the dialysis treatment and examples of these devices are the AccuGage™Vessel Caliper produced and supplied by the company Accumed Systems, Inc.; the product Easy Radial developed by Dr. M. Galli and manufactured and supplied by the company Blue Medical Devices; the product RadiStop™ Radial Compression System, produced and supplied by the company RadiMedical Systems AB; the product Radstat™ hemostasis device, developed by M. E. Arrowroot P.A.C., the product Hasin hemostasis device, developed by Professor Y. Hasin, the product Stepty®P hemostasis device, produced and supplied by the company Nichiban Corporation, Ltd, the product Adapty, a modification of Stepty®P hemostasis device, developed by Dr. Hideaki Sakai. Additional examples of prior art compressive appliances or hemostasis establishing devices are described in US 5 512 056, US 5 133 734, and WO 98 46144.

An object of the present invention is to provide an improved device as compared to the number of compressive appliances already known improving the mechanical compression technique by preventing congestion and ischemia by eliminating the risk of stasis and at the same time, releasing nurse time and improving the comfort for the patient.

A particular feature of the present invention relates to the application of the device, as the device may readily be applied providing the correct pressure to the area or the site at which compression is to be applied for establishing hemostasis.

A particular advantage obtained by the teachings of the present invention relates to the fact that the device according to the present invention for establishing hemostasis is of a low cost structure allowing the device, which is brought into contact with a patient or person, may be implemented as a disposable device, thereby preventing any risk of cross contamination.

The above object, the above advantage, the above feature together with numerous other objects, advantages and features which will be evident from the below disclosure of advantageous and preferred embodiments of the device according to the present invention are according to the present invention obtained by a device for establishing hemostasis of an open artery and/or a vein of an extremity of a person through mechanically compressing an area of said extremity at said open artery and/or vein, said device comprising: a pair of elongated strips comprising a first strip and a second strip, said first strip being made of a first, flexible and bendable material and/or being configurated in a curved shape and defining a length exceeding the width of said extremity by a factor of no less than 1.5, said first strip defining opposite first and second ends, said second strip being made of a second, flexible and bendable material and defining a length exceeding the width of said extremity by a factor of no less than 1.5, said second strip defining opposite third and fourth ends, said first strip being connected to or connectable to said second strip at said first end and said third end, respectively, through a hinge connection or through co-operating and interlocking connectors, said first strip being provided with a first connector element at said second end thereof and second strip being provided with a second connector element or a plurality of second connector elements at said fourth end thereof for co-operating with said first connector element of said first strip, and
a liquid absorbing pad arranged around the centre of said first strip or alternatively said second strip and for positioning at said area of said extremity at said open artery and/or vein and being supported relative to said first strip or alternatively said second strip by a compressible or flexible element including or co-operating with an indicator element for indicating the application of a specific pressure to said area of said extremity at said open artery and/or vein by said pad, and said first and second strips being positionable circumferentially encircling said extremity for applying said specific pressure to said area at said open artery end or vein by the adjustment of said locking between said first strip and said second strip through said first and second connector elements.

According to the basic teachings of the present invention, the device for establishing hemostasis includes a pair of strips which are in the intended application of the device positioned circumferentially encircling the extremity, such as the wrist of a patient or person. The one strip presses the pad into contact with the application site, such as the wounds or the open artery and/or the open vein at the wrist of the patient or person and due to the presence of the indicator included in the device according to the present invention, the person applying the device readily adjusts the pressure applied to the application area or application site by adjusting the co-operating first and second connector elements and at the same time monitoring the pressure reading of the indicator.

As will be discussed in greater details below, the indicator may be implemented in numerous embodiments including any mechanical pressure indicator appliance including bending, stretching, compression registering or reading devices etc.

According to the presently preferred embodiment of the device according to the present invention, the first material of the first strip is identical to the material of the second strip allowing the two strips to be made from a single material, such as a plastics material, e.g. a skin compatible material, such as PE, PP, ABS or any other equivalent plastics material. The connection between the first and the second strips may be established through interlocking connectors, however, according to the presently preferred embodiment of the device according to the present invention, the connection between the first and the second strip is established by an integral hinge or a foil hinge of the strip material from which the first and the second strips are produced. Although plastics materials are presently contemplated to be preferred, different materials such as aluminium or any other corrosion resistant material may be used or used in combination with plastics materials, in particular for providing additional strength or specific mechanical support of one of the two strips or other components or elements of the device according to the present invention.

The locking elements by means of which the first and second strips be locked together circumferentially encircling the extremity of the person may be constituted by any locking fixtures, such as catches, burr or Velcro® fixtures, flexible bands to be tied round a pair of arresting pins or similar elements, however, according to a presently preferred embodiment of the device according to the present invention, the first connector element is constituted by a pin cut from the first strip and the second locking element or each of the plurality of the second locking elements are constituted by recesses or holes of the extension of the second strip.

The indication of the pressure applied by the pad at the application site such as the wound or open artery or open vein at the wrist of a patient or person may, as already stated, be provided by any indicator element capable of reading and displaying the compression or the pressure applied by the pad. According to a particular elegant and advantageous embodiment of the device according to the present invention, the compressible or flexible element is constituted by one or more additional pad elements supporting the liquid absorbing pad. By combining the indicator with the liquid absorbing pad in a pad assembly, a simple, lightweight and cheap structure is obtained providing a disposable device. The additional pad elements constituting the indicator may include a non-compressible pad and a compressible pad, may provide the indications through the reduction of the thickness of the compressible pad to a specific ratio, such as 50% of the thickness of the compressible pad in uncompressed state or alternatively through compression of the compressible pad to a thickness corresponding to the thickness of the non-compressible pad or the thickness of the liquid absorbing pad.

According to an alternative embodiment which may advantageously be implemented in embodiments in which the strips are cast or moulded, the indicator of the device according to the present invention constituted by the compressible or flexible element, is advantageously constituted by a spring element providing the indications through the positioning of an indicator, such as an end of the spring element relative to a pre-set marking.

An alternative embodiment of the device according to the present invention is provided by implementing the second strip as a flexible or bendable plastics band of a woven or non woven material which is readily tightened round the first connector element of the first strip while positioning the two strips circumferentially encircling the extremity of the patient or person in question.

As already mentioned above with reference to the discussion of the provision of the indicator as a spring element of a cast or moulded strip, the first strip may be pre-cast in a specific curved configuration and also cast with corrugations or with integral stiffening elements for ensuring that the first strip be kept in its intentional specific curved configuration.

Dependant on the actual application of the device according to the present invention for circumferentially encircling the wrist or alternatively the leg or any other extremity of a person, the length of the first and/or second strip as compared to the width of the extremity, may vary from the estimated lower limit of 1.5-5.0, such as between 1.5 and 2.0 or vary between 1.5 and 1.6, 1.6-1.7, 1.7-1.8, 1.8-1.9, 1.9-2.0, 2.0-2.5, 2.5-3.0, 3.0-3.5, 3.5-4.0, 4.0-5.0.

In order to prevent that the liquid absorbing pad, which is brought into contact with the application site, i.e. the wound and or the opening into the vein of the patient, is slipping on the skin surface of the patient or person, e.g. due to the presence of moisture or blood, the liquid absorbing pad is preferably covered by a perforated high frictional foil.

The present invention is now to be further described with reference to the drawings in which:
Fig. 1 is a perspective and schematic view of a first and presently preferred embodiment of a device according to the present invention for establishing hemostasis of an open artery or vein of an extremity such as the forearm or wrist of a person,
Fig. 1a is a perspective and schematic view similar to the view of Fig. 1 of a modified, two part form of the presently preferred embodiment of the device according to the present invention shown in Fig. 1,
Fig.2 is a perspective and schematic view similar to the views of Figs. 1 and 1a of a second embodiment of the device according to the present invention,
Fig. 3 is a perspective and schematic view similar to the views of Figs. 1 and 1a of a third embodiment of the device according to the present invention,
Fig. 4 is a perspective and schematic view similar to the views of Figs. 1 and 1a of a fourth embodiment of the device according to the present invention,
Fig. 5 is a perspective and schematic view similar to the views of Figs. 1 and 1a of a fifth embodiment of the device according to the present invention,
Fig. 6 is a perspective and schematic view of the application and intentional use of the third embodiment of the device according to the present invention also shown in Fig. 3 as the device is applied to the wrist or forearm of the person,
Fig. 7 is a perspective and schematic view similar to the view of Fig. 6 of the application and intentional views of the fourth embodiment of the device according to the present invention also shown in Fig. 4,
Fig. 8 is a vertical sectional view illustrating the application or intentional use of the second embodiment of the device according to the present invention also shown in Fig. 2,
Fig. 9 is a vertical sectional view similar to the view of Fig. 8 of a further modified embodiment of the device according to the present invention,
Fig. 10 is a partly sectional, perspective and schematic view of a compression applying pad assembly of the device according to the present invention,
Figs. 11a, 11b and 11c are schematic views illustrating a particular feature of the pad assembly shown in the application and intentional use of the device according to the present invention for monitoring the application of a specific pressure to the compression site or area at the wrist or other extremity of a person.
Figs. 12a, 12b and 12c are schematic views similar to the views of Figs. 11a, 11b and 11c illustrating a different implementation of a monitor for monitoring the application of the specific pressure to the compression site or area,
Fig. 13 is a perspective and schematic view of a still further modified embodiment of the device according to the present invention including a further implementation of the monitor for monitoring the application of the specific pressure to the compression site or area at the wrist or other extremity of a person, and
Fig. 14a and 14b are vertical sectional views of the embodiment shown in Fig. 13 and illustrating the monitoring of the application of the specific pressure to the compression site or area.

The present invention relates in general to a refined and novel technique of establishing a specific pressure at an area or a site of an extremity of a person or a mammal, which area or site includes an open artery or vein. The present invention relates in particular to a device for use in specific surgical circumstances, such as the treatment of an individual after the individual has been subjected to a dialysis treatment. In a dialysis treatment, the artery and the vein of the wrist of the individual or person of one of the arms of the individual or person in question are opened for allowing the staff operating the dialysis apparatus to direct the blood through the dialysis apparatus from the artery of the wrist of the person or individual to the artery of the person or individual. After the dialysis treatment has been finalised, the open artery and vein are to be compressed for establishing hemostasis before the person or individual is allowed to leave the dialysis clinic or hospital.

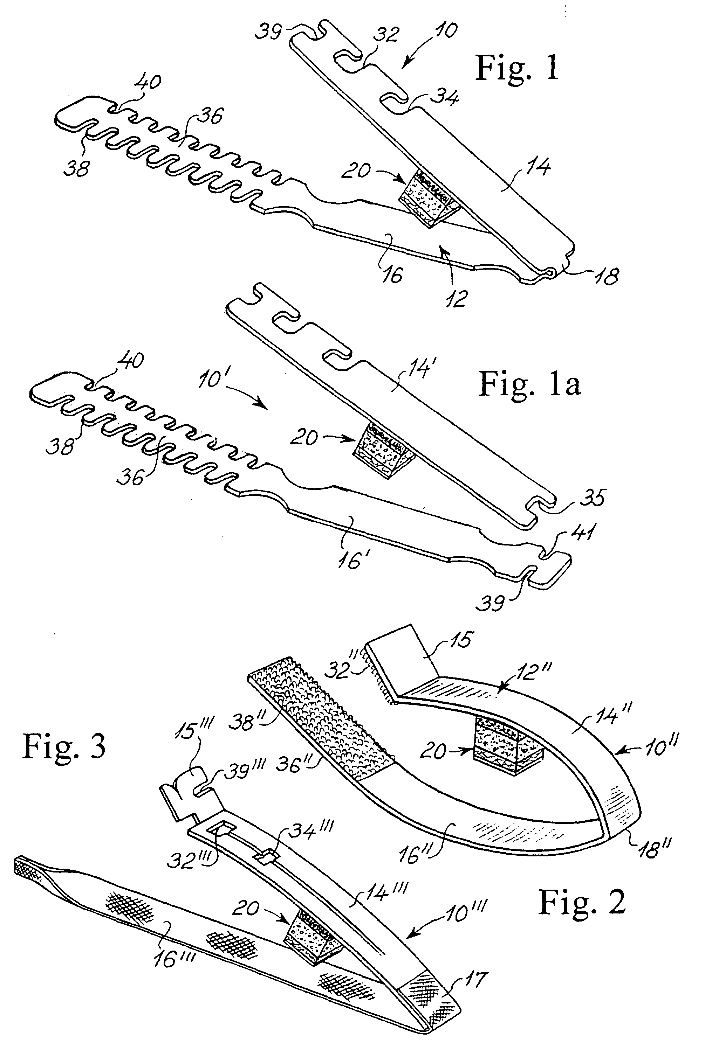

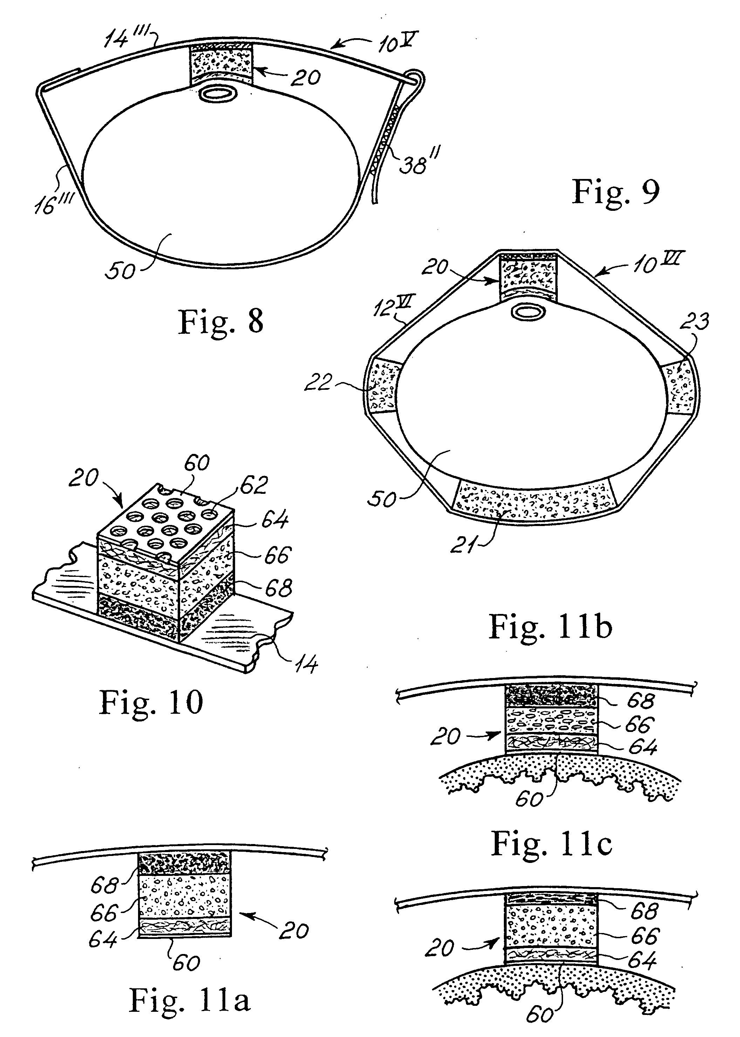

In Fig. 1, a first and presently preferred embodiment of the device according to the present invention is shown, designated the reference numeral 10 in its entirety. The device according to the present invention comprises a strip of a plastics material such as a strip of PE, PP or ABS or similar biologically acceptable plastics material or alternatively, aluminium or other non-corrosive metal materials. The strip 12 is punched or cast in a two-part configuration comprising two strips 14 and 16. Each of these strips define opposite ends and are connected integrally to one another through a film, foil or integral hinge designated the reference numeral 18. At a central position, the strip 14 is supporting a pad assembly 20 facing the strip 16 which assembly is to be described in greater details below with reference to Figs. 10 and 11a, 11b and 11c.

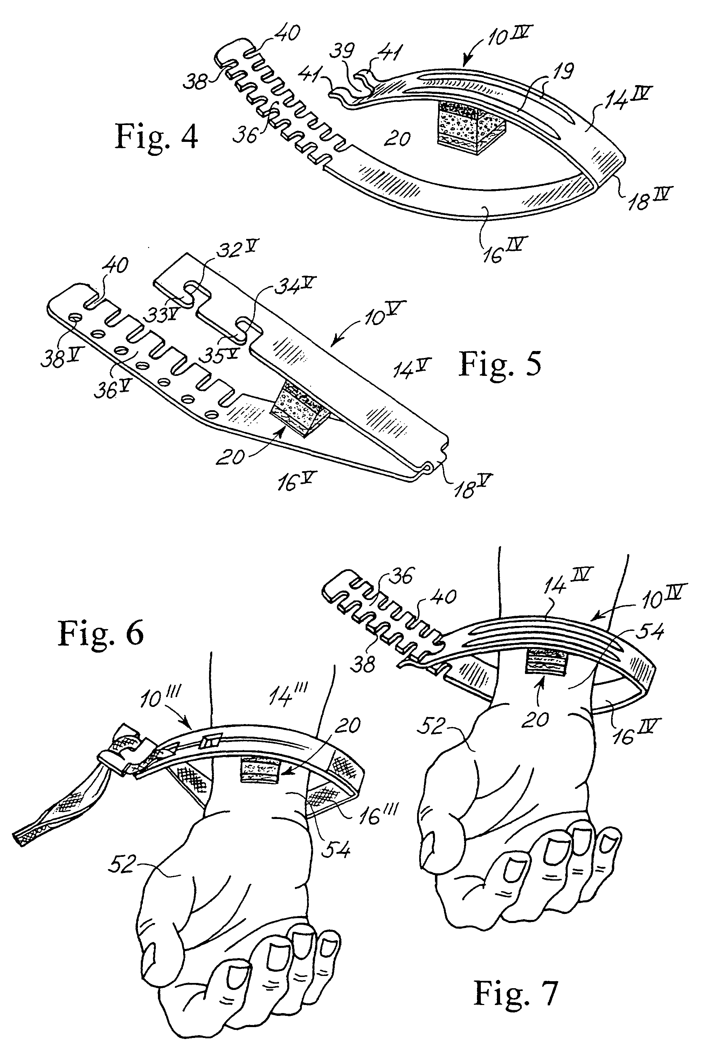

The strip 14 is at its end opposite to the end connected to the strip 16 through the film hinge 18 provided with two side recesses 30 and 32 and an end recess 34. Similarly, the strip 16 is at its one end opposite to the end connected to the strip 14 through the hinge 18 provided with an extension in which nine pairs of side recesses are provided, one of the side recesses from the one side being designated the reference numeral 38 and a side recess at the opposite side of the extension 36 being designated the reference numeral 40. The recesses 38 and 40 serve the purpose of allowing one of the recesses 32 or 34 to be received by the side recesses, such as the pair 38 and 40, as the device 12 is applied to the wrist or other extremity of a person as is illustrated in Figs. 6 and 7. The presence of nine pairs of recesses 38, 40 and the presence of two side recesses 32 and 34 of the strip 16 and the strip 14, respectively, allows the device 10 to accommodate a wrist of a person, such as a child having a slim wrist and also a large wrist of e.g. a male.

In Fig. 1a, a modified two-part form of the above-described presently preferred embodiment 10 shown in Fig. 1 is illustrated. In the various figures, components or elements identical to components or elements, respectively, described above, are designated the same reference numerals as used in a previous figure, whereas components or elements serving the same purpose as an element or component described with reference to a previous figure, is designated the same figure, however added a signature, i.e. a marking identifying the geometrical difference from the previously described component or element. In Fig. 1a, the strips 14' and 16' differ from the strips 14 and 16 described above with reference to Fig. 1 in that the hinge 18 is omitted and the strip 14 is provided with an end recess 35 similar to the recess 34 provided at the opposite end of the strip 14 and in that the strip 16' at its one end opposite to the extension 36 is provided with side recesses 39 and 41 serving the purpose of allowing the end recess 34 of the strip 14' to be received for connecting the strips 14' and 16' together in a joint connection similar to a hinge connection.

In Fig. 2, a second embodiment of the device according to the present invention is shown designated the reference numeral 10" in its entirety. The strip 12" shown in Fig. 2 is of a pre-cast configuration differing from the above described first and the above described modified two-part form of the first embodiment in that the recess fixture of the strip 14 relative to the strip 16 by means of the co-operating recesses 32 and 34 of the strip 14 and the recesses 38 and 40 of the strip 16 are substituted by a pair of burr or Velcro® fixtures 32" and 38" provided at an extension 15 of the strip 12" and the extension 36" of the strip 16".

In Fig. 3, a third embodiment of the device according to the present invention is shown designated the reference numeral 10''' in its entirety. The device 10''' differs from the above-described first embodiment 10 shown in Fig. 1 and the above-described embodiment 10" shown in Fig. 2 in that the strip 16 is made from a woven material constituting a woven band or strip as distinct from the plastic foil strips 12 and 12" of the devices 10 and 10", respectively. In Fig. 3, the strip or band 16''' is glued or otherwise adhered to the strip 14''' by a turned-in part 17 fixated to the one end of the strip 14''' opposite to the free end 15''' to which the band 16' is fixated after the strip or band has been threaded through one of the holes 32''' or 34''' as is shown in greater details in Fig. 6.

In Fig. 4, a fourth embodiment of the device according to the present invention is shown designated the reference numeral 10^{iv} in its entirety. Like the above-described second embodiment 10" shown in Fig. 2, the fourth embodiment 10^{iv} shown in Fig. 4 is made from an integrally cast plastic component defining the integrally connected strips 14^{iv} and 16^{iv} which are connected through the integral hinge or film hinge 18^{iv}. The strip 14^{iv} is, as is shown in Fig. 4, provided with corrugations 19 providing increased stiffness to the top strip 14^{iv} as compared to the strip 16^{iv} and at the same time configurates the strip 14^{iv} in a specific curved shaped as is illustrated in Fig. 4. the outer end of the strip 14^{iv} is provided with a recess 39 defining two upwardly turned lathes 41 serving the purposes of co-operating with the side recesses 38 and 40 of the extensions 36 of the strip 16^{iv}.

In Fig. 5, a fifth embodiment of the device according to the present invention is shown designated the reference numeral 10^{v} in its entirety and differing from the above-described first embodiment 10 shown in Fig. 1 in that the recesses 32^{v} and 34^{v} are provided as mirror images of the recesses 32 and 34 shown in Fig. 1 and in that the end recess 39 is omitted. In the strip 16^{v} which is pre-shaped into a bent configuration, the extension 36^{v} of the strip 16^{v} is provided with one row of recesses 40 and corresponding to the recesses 40, a plurality of through-going holes 38^{v} are provided serving the purpose of receiving one of the protruding pins 33^{v} and 35^{v} defined by the recesses 32^{v} and 34^{v}, respectively.

In Fig.6, the application or intentional use of the third embodiment 10''' of the device according to the present invention is illustrated in greater details. In Fig. 6, the forearm 50 of a person is shown together with the hand 52 of the same person. At the wrist 54 of the person, the device 10''' is applied. It is contemplated that the individual having the arm 50, has previously been subjected to a treatment or alternatively an accident producing an opening into the artery and/or the vein at the wrist 54 of the forearm 50.

An example of the usage of the device according to the present invention is the establishing of hemostasis in the openings into the artery and the vein of a person being treated in a dialysis apparatus in which case the artery and the vein of the person are opened for allowing the blood to be guided through the dialysis apparatus. After the dialysis treatment, the openings or wounds to the artery and the vein are to be closed and contrary to the manual pressing of the area around the artery opening and the vein opening together, as it has previously been practised in most clinics and hospitals, the device according to the present invention is readily usable for this purpose. According to a particular feature of the device according to the present invention, the strip 14 and also the strip 16 are fairly broad for ensuring that the only point of contact between the strip 14 and the forearm of the persons is at the pressure application site, i.e. at the site of contacting the pressure pad assembly 20 to the openings into the artery and/or the vein of the person. The strip 14 and similarly the strip 16 define a span exceeding the width of the wrist of the individual.

As is seen from Fig. 6, the device 10"' apart from contact with the application site for applying pressure to the openings into the artery and the vein through the pad assembly 20, the device only contacts the forearm at the opposite side of the forearm through the strip or band 16.

According to a particular feature of the device implemented in accordance with the teachings of the present invention, the application of the device by a person, such as a nurse, is easily carried out and at the same time safely done while providing a specific pressure such as a pressure of the order of 300-400g/cm² at the site of applying the pad assembly 20 as a component of a device, in particular the pad assembly includes an indicator informing the person applying the device to the forearm or the wrist 54 about the correct pressure applied to the application site through the pad 20. This feature is to be discussed and described in greater details below with reference to Figs. 11-14. The fixation of the device 10"' as shown in Fig. 6 is easily accomplished by simply twisting the strip or band 16 through the recesses and slits of the end parts 15"' after the strip or band 16 has been threaded through one of the holes 32"' and 34"', in the application shown in Fig. 6 through the hole 32"'.

In Fig. 7, the application of the fourth embodiment 10^{iv} shown in Fig. 4 is illustrated in a view similar to the view of Fig. 6. In Fig. 7, no detailed description of the extremity of the person is given as the only feature to be referred to in Fig. 7 relates to the possibility of adjusting the width and also the height of the device 10^{iv} circumferentially encircling the wrist 54 by positioning the two lathes 41 in a specific pair of recesses 38, 40 of the extension 36 of the strip 16^{iv} for providing the intentional application of a specific pressure by the pad assembly 20 to the open artery and/or vein for establishing hemostasis.

In Fig. 8, a vertical sectional view is shown illustrating a modified version 10^{v} of the third embodiment 10''' shown in Figs. 3 and Figs. 6 and differing from the third embodiment 10''' in that the fixation of the strip or band 16''' is provided through a burr or Velcro fixture of the strap 16''' itself, i.e. a fixture similar to the fixture shown in Fig. 2.

In Fig. 9, a further variant of the device according to the present invention is shown designated the reference numeral 10^{vi}. The device 10^{vi} shown in Fig. 9 differs from the above-described embodiments in that the strips 14 and 16 are combined into a single strap 12^{vi} supported by the pressure applying pad assembly 20 and further three additional pads 21, 22 and 23 positioned in an orthogonal setup relative to the arm 50. The pressure applying pad 20 is of the configuration to be described below for applying a specific predetermined pressure to the application site and the additional pads 21, 22 and 23 together with the strap 12^{vi} which constitutes an elastic strap accommodate any excessive pressure provided the arm 50 is of a somewhat larger periphery than the normal size of the forearm to which the device 10^{vi} is adapted, thereby establishing a multipad pressure system in combination with an elastic strap for establishing a specific pressure through the pad assembly to the application site for establishing hemostasis.

In Fig. 10, the pad assembly 20 is shown in greater details including a total of four layers and a perforated high frictional foil 60, in which a plurality of holes or apertures 62 are provided. The foil 60 is of a skin compatible material such as siliconised polyester. Below the foil 60, a liquid and blood absorbing pad 64 is provided made from a skin gentle material and also a highly absorbant material, such as a polyamide foam, preferably hemostatically impregnated. Furthermore, the pad 20 includes two parts 66 and 68 which in an alternative embodiment may be constituted by a single pad or configurated differently, as will be described below.

I the presently preferred embodiment, the pad 66 is a PU foam pad of a stiffness similar to the polyamide absorbing pad 64. Below the PU foam pad 66, a compressible pad made from a different PU foam material as compared to the foam material of the pad 66 is provided, which pad constitutes an indicator for informing the person applying the device according to present invention to the forearm of a patient or person about the pressure applied to the application site.

It has been realised that hemostasis is established by use of a contact area measuring 4cm² provided a pressure of 300-400, preferably 350-400g be applied to the 4cm² contact area. Consequently, the pressure to be applied to the application site is preferably of the order of 90-100g/cm². For indicating to the person applying the device, the indicator pad 68 is adapted to be compressed to 50% of its initial height provided a pressure of 400g/4cm² equivalent to 100g/cm² be applied to the assembly 20. Consequently, as the person applying the device tightens the device round the wrist of the person as is illustrated generally in Figs. 6 and 7, the person monitors the compression of the indicator pad 68 and tightens the device to such an extent that the pad 68 is compressed to 50% of its initial height, which reduction is easily perceived by the person applying the device.

In Fig. 10, the indicator pad 68 is illustrated in a dark colour which is contemplated to be advantageous for clearly indicating to the person applying the device that the dark coloured pad 68 constitutes the indicator pad which is to be monitored for monitoring the compression of the pad to 50% of its original height. In an alternative embodiment, the pad 66 and 68 are shifted and the indicator included in the pad assembly 20 is provided by the central pad as the indication is established by a compression of the indicator pad to a height identical to the height of the adjacent non-compressible pad.

Different configurations of the indicator system may of course be deduced based on the compression of a specific pad element, the compression of which is monitored in relation to its original height or in relation to an adjacent element constituting a measure of the intentional compression of the compressible indicator pad. In Figs. 11a, 11b and 11c, the pad assembly 20 is shown in greater details in an uncompressed state shown in Fig. 10a. In Fig. 11c, the indicator pad is compressed to 50% of its initial height shown in Fig. 11a and therefore, the pad assembly 20 is compressed to its intentional compressed state applying approximately 300-400g/cm² equivalent to approximately 75-100g/cm². n Fig. 11b. a differently configurated indicator system in which the central pad 66 constitutes the compressible pad, which as compared to the initial and uncompressed state shown in Fig. 11a is compressed to a height identical to the height of the indicator pad 68.

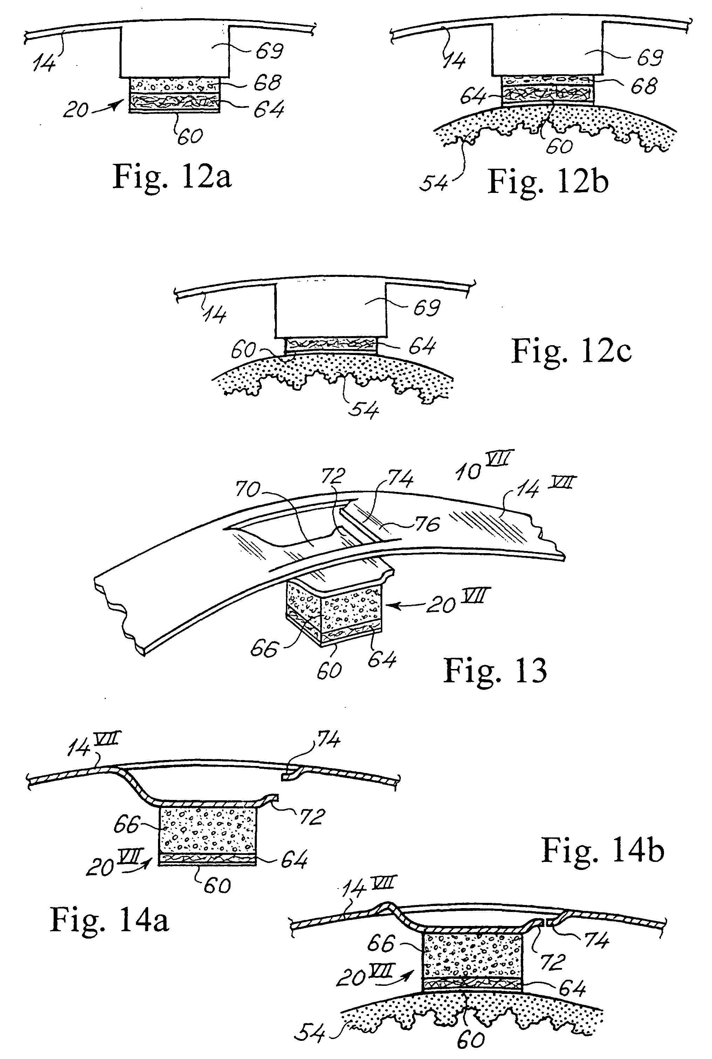

In Figs. 12a-12c, a different indicator system is shown, which system includes a differently configurated pad assembly 20' including the perforated high frictional foil 62, the absorbing pad 64 and a single indicator pad 68'. The pad assembly 20' shown in Fig. 12a-12c is included within a circumferential housing or hidden behind a wall 69 constituting an integral part of the strip 14 and protrudes from the lower edge of the wall or the lower rim of the circumferential housing 69 in the uncompressed state shown in Fig. 12a. As the device including the pad assembly 20' is applied to the skin area or skin site of the person, e.g. as illustrated in Figs. 6 and 7, the compressible indicator pad 68' is compressed as shown in Fig. 12b. At the time the boarder line between the liquid and blood absorbing pad 64 and the compressible indicator pad 68' be hidden behind the wall 69 as shown in Fig. 12c, the intentional pressure is provided at the application site of the order of 300-400g/cm² equivalent to 70-100g/cm².

In Fig. 13, a further embodiment of the strip 14^{vii} of a further embodiment 10^{vii} of the device according to the present invention is shown. Whereas the pad assembly supporting strips 14, 14', 14", 14''', 14^{iv} and 14^{v} are integral elements of a planar configuration or as shown in Figs. 2 and 3 curved configuration, the embodiment shown in Fig. 13 includes a central spring element 70 depending from the curved strip 14^{vii} and providing a support for the pad assembly 20^{vii} which differs from the above-described pad 20 in that the pad assembly 20^{vii} includes the liquid and blood absorbing pad 64' covered by the perforated high frictional foil not shown in Fig. 13 and further, a support pad 66' of a basical non-compressible PU material. The downwardly depending spring 70 is provided with a free outer edge 72 which is positioned juxtaposed the free edge 74 of a minor downwardly depending plate element 76.

The edges 72 and 74 serve as indicators for informing the person applying the device 10^{vii} to the forearm or the wrist of a person as is illustrated in Figs. 6 and 7, as the edge 72 is in an unbiased or uncompressed state as shown in Fig. 14a, positioned in spaced apart relationship relative to the edge 74, whereas the edges 72 and 74 as shown in Fig. 14b are positioned closely adjacent one another provided the spring 70 is subjected to biasing force corresponding to the intentional pressure to be applied by the pad assembly 20^{vii} to the application site, i.e. a pressure of the order of 75-100g/cm². Provided an excessive pressure be applied to the application site, the edge 72 is raised above the adjacent edge 74 indicating to the person applying the device 10^{vii} that an excessive pressure is generated.

### Example

The presently preferred embodiment of the device according to the present invention shown in Fig. 1 was made from the following components: The strip 12 comprising the two strips 14 and 16 was made from PET of a thickness of 1.5mm. The strip 14 had a length of 400.0mm and a width of 22mm and the strip including the extension 36 had a length of 238.2mm. The hinge 18 was an integral film hinge. The pair of recesses 38 and 40 amounted to 9 as shown in Fig. 1 and the width of each of the recesses 38 and 40 was 5.0mm and the depth of each of the recesses 38 and 40 was 6.5mm. The end recess 39 of the strip 14 measured: depth 6.5mm and width 5.0mm. The side recesses 32 and 34 defined a lateral depth of 12mm and a longitudinal extension of 12mm. The width of the opening at the edge of the strip 14 defined by each of the recesses 32 and 34 measured 4mm.

The pad assembly 20 was measuring length x width = 4.84cm² and a total height of 1.82cm. The perforated high friction foil 60 was made from a PU foamed foil of a thickness of 0.2mm and each of the apertures 62 had a diameter of 3.5mm. The liquid absorbing pad 64 was made from hemostatically impregnated polyamide foam and defined a height of 3.5mm. The non-compressible pad 66 was made from PU foam and defined a height of 9.0mm. The compressible indicator pad 68 was made from PU foam and defined in its initial uncompressed state a height of 5.5mm.

Although the present invention has been described above with reference to presently preferred embodiments, the invention is by no means limited to the above-described advantageous embodiments as the features described in the various embodiments are readily combinable with one another and further modifications or changes in the above-described embodiments, elements or components obvious to a person having ordinary skill in the art are to be considered part of the present invention as defined in the appending claims.

## Claims

1. A device for establishing hemostasis of an open artery and/or a vein of an extremity (52, 54) of a person through mechanically compressing an area of said extremity at said open artery and/or vein, said device (10) comprising:
a pair of elongated strips (14, 16) comprising a first strip (14) and a second strip (16), said first strip being made of a first, flexible and bendable material and/or being configurated in a curved shape and defining a length exceeding the width of said extremity by a factor of no less than 1.5, said first strip (14) defining opposite first and second ends, said second strip (16) being made of a second, flexible and bendable material and defining a length exceeding the width of said extremity by a factor of no less than 1.5, said second strip (16) defining opposite third and fourth ends, said first strip (14) being connected to or connectable to said second strip at said first end and said third end, respectively, through a hinge connection (18) or through co-operating and interlocking connectors (35, 39, 41; 17), said first strip (14) being provided with a first connector element (32, 34, 39) at said second end thereof and second strip (16) being provided with a second connector element (36, 38, 40) or a plurality of second connector elements at said fourth end thereof for co-operating with said first connector element of said first strip (14), and
a liquid absorbing pad (20, 64) arranged around the centre of said first strip (14) or alternatively said second strip (16) and for positioning at said area of said extremity at said open artery and/or vein and being supported relative to said first strip or alternatively said second strip by a compressible or flexible element (66; 68) including or co-operating with an indicator element (68; 64; 69) for indicating the application of a specific pressure to said area of said extremity at said open artery and/or vein by said pad, and said first and second strips (14, 16) being positionable circumferentially encircling said extremity (53, 54) for applying said specific pressure to said area at said open artery end or vein by the adjustment of said locking between said first strip and said second strip through said first and second connector elements.

2. The device according to claim 1, said first material of said first strip being identical to said second material of said second strip and being a plastics material, and said connection (18) between said first and second strips being constituted by an integral hinge or a foil hinge of said strip material (12).

3. The device according to any of the claims 1 or 2, said first connector element (32, 34, 39) being constituted by a pin cut from said first strip (14) and each of said plurality of second locking elements (36, 38, 40) being constituted by a recess or hole of said extension of said second strip.

4. The device according to any of the claims 1-3, said compressible or flexible element being constituted by one or more additional pad elements (66, 68) supporting said liquid absorbing pad (64).

5. The device according to claim 4, said additional pad elements (66, 68) including a non-compressible pad (68) and a compressible pad (66) and said compressible pad (66) providing said indication through the reduction of the thickness of said compressible pad to a specific ratio such as 50% of the thickness of said compressible pad in uncompressed state or alternatively through compression of said compressible pad to a thickness corresponding to the thickness of said non-compressible pad (68) or the thickness of said liquid absorbing pad (64).

6. The device according to any of the claims 1-3, said compressible or flexible element being constituted by a spring element (70) providing said indication through the positioning of an indicator (72) of said spring element relative to a preset marking (74).

7. The device according to any of the claims 1-6, said second strip being constituted by a flexible or bendable plastics band of a woven or non-woven material.

8. The device according to any of the claims 1-7, said first strip being pre-cast in a specific curved configuration.

9. The device according to any of the claims 1-8, said liquid absorbing pad (64) being covered by a perforated high frictional foil (60).

## Patentansprüche

1. Vorrichtung zum Erzeugen einer Hämostase einer offenen Arterie und / oder Vene einer Extremität (52, 54) einer Person durch mechanische Kompression eines Bereichs der genannten Extremität bei der genannten offenen Arterie und / oder Vene, wobei die genannte Vorrichtung (10) Folgendes umfasst:
ein Paar gestreckter Streifen (14, 16), das einen ersten Streifen (14) und einen zweiten Streifen (16) umfasst, wobei der genannte erste Streifen aus einem ersten, flexiblen und biegbaren Material hergestellt ist und / oder in gekrümmter Form ausgestaltet ist und eine Länge vorgibt, die die Breite der genannten Extremität um einen Faktor von nicht weniger als 1,5 übersteigt, wobei der genannte erste Streifen (14) ein erster und zweites, gegenüberliegendes Ende vorgibt, wobei der genannte zweite Streifen (16) aus einem zweiten, flexiblen und biegbaren Material hergestellt ist und eine Länge vorgibt, die die Breite der genannten Extremität um einen Faktor von nicht weniger als 1,5 übersteigt, wobei der genannte zweite Streifen (16) ein drittes und viertes, gegenüberliegendes Ende vorgibt, wobei der genannte erste Streifen (14) mit dem genannten zweiten Streifen am genannten ersten Ende beziehungsweise am genannten dritten Ende durch eine Scharnierverbindung oder mittels wechselwirkender und verriegelnder Verbinder (35, 39, 41, 17) verbunden oder verbindbar ist, wobei der genannte erste Streifen (14) an seinem genannten zweiten Ende mit einem ersten Verbindungselement (32, 34, 39) versehen ist und der zweite Streifen (16) an seinem genannten vierten Ende mit einem zweiten Verbindungselement (36, 38, 40) oder einer Vielzahl zweiter Verbindungselemente zur Wechselwirkung mit dem genannten ersten Verbindungselement des genannten ersten Streifens (14) versehen ist, und
ein Flüssigkeit absorbierendes Pad (20, 64), das im Bereich des Zentrums des genannten ersten Streifens (14) oder alternativ des genannten zweiten Streifens (16) angeordnet ist und zur Anordnung im genannten Bereich der genannten Extremität bei der genannten offenen Arterie und / oder Vene bestimmt ist und das relativ zum genannten ersten Streifen oder altemative zum genannten zweiten Streifen durch ein kompressibles oder flexibles Element (66, 68) gehalten wird, das ein Anzeigeelement (68, 64, 69) zur Anzeige der Anwendung eines spezifischen Drucks durch das Pad auf den Bereich der genannten Extremität bei der genannten offenen Arterie und / oder Vene beinhaltet oder mit diesem zusammenwirkt, und wobei der genannte erste und zweite Streifen (14, 16) die genannte Extremität (53, 54) umfänglich umfassend anordbar sind, um den genannten spezifischen Druck auf den genannten Bereich an der genannten offenen Arterie und / oder Vene durch Anpassung der genannten Verriegelung durch die genannten ersten und zweiten Verbindungselemente zwischen dem genannten ersten Streifen und dem genannten zweiten Streifen anzulegen.

2. Vorrichtung gemäß Anspruch 1, wobei das genannte erste Material des genannten ersten Streifens mit dem genannten zweiten Material des genannten zweiten Streifens identisch und Kunststoffmaterial ist, und wobei die Verbindung (18) zwischen dem genannten ersten und zweiten Streifen durch ein integriertes Scharnier oder ein Filmscharnier aus dem genannten Streifenmaterial (12) gebildet ist.

3. Vorrichtung gemäß einem der Ansprüche 1 oder 2, wobei das genannte erste Verbindungselement (32, 34, 39) durch einen in den genannten ersten Streifen (14) geschnittenen Dorn gebildet wird und jedes aus der genannten Vielzahl der zweiten Verbindungselemente (36, 38, 40) durch eine Ausnehmung oder ein Loch der genannten Verlängerung des genannten zweiten Streifens gebildet wird.

4. Vorrichtung gemäß einem der Ansprüche 1 - 3, wobei das genannte kompressible oder flexible Element durch ein oder mehr zusätzliche Pad-Elemente (66, 68), die das genannte Flüssigkeit absorbierende Pad (64) halten, gebildet wird.

5. Vorrichtung gemäß Anspruch 4, wobei die genannten zusätzlichen Pad-Elemente (66, 68) ein nicht-kompressibles Pad (68) und ein kompressibles Pad (66) umfassen, und das genannte kompressible Pad (66) die genannte Anzeige durch eine Dickenverringerung des genannten kompressiblen Pads auf ein spezifisches Verhältnis, wie 50 % der Dicke des genannten kompressiblen Pads im nicht-komprimierten Zustand, oder alternativ durch Kompression des genannten kompressiblen Pads auf eine Dicke, die der Dicke des genannten nicht-kompressiblen Pads (68) oder der Dicke des genannten Flüssigkeit absorbierenden Pads (64) entspricht, bereitstellt.

6. Vorrichtung gemäß einem der Ansprüche 1 bis 3, wobei das genannte kompressible oder flexible Element durch ein Federelement (70) gebildet wird, das die genannte Anzeige durch Positionierung eines Indikators (72) des genannten Federelements relative zu einer vorgegebenen Markierung (74) bereitstellt.

7. Vorrichtung gemäß einem der Ansprüche 1 bis 6, wobei der genannte zweite Streifen durch ein flexibles oder biegbares Kunststoffband aus gewebtem oder nicht-gewebtem Material gebildet wird.

8. Vorrichtung gemäß einem der Ansprüche 1 bis 7, wobei der genannte erste Streifen in einer spezifisch gekrümmten Ausgestaltung vorgefertigt ist.

9. Vorrichtung gemäß einem der Ansprüche 1 bis 8, wobei das genannte Flüssigkeit absorbierende Pad (64) durch eine perforierte, hoch reibschlüssige Folie (60) bedeckt ist.

## Revendications

1. Dispositif pour réaliser l'hémostase d'une artère et/ou d'une veine ouverte d'une extrémité (52, 54) d'une personne par compression mécanique d'une zone de ladite extrémité au niveau de ladite artère et/ou veine ouverte, ledit dispositif (10) comprenant :
une paire de languettes longitudinales (14, 16), comprenant une première languette (14) et une deuxième languette (16), ladite première languette étant faite d'un premier matériau flexible et incurvable et/ou étant configurée selon une forme courbe et définissant une longueur excédant la largeur de ladite extrémité d'un facteur non inférieur à 1,5, ladite première languette (14) définissant une première et une deuxième extrémités opposées, ladite deuxième languette (16) étant faite d'un deuxième matériau flexible et incurvable et définissant une longueur excédant la largeur de ladite extrémité d'un facteur non inférieur à 1,5, ladite deuxième languette (16) définissant une troisième et une quatrième extrémités opposées, ladite première languette (14) étant reliée ou reliable à ladite deuxième languette au niveau respectivement de ladite première extrémité et de ladite troisième extrémité, par un joint à charnière (18) ou par des raccords coopérant et se verrouillant mutuellement (35, 39, 41 ; 17), ladite première languette (14) étant munie d'un premier élément de raccord (32, 34, 39) au niveau de ladite deuxième extrémité de celle-ci, et la deuxième languette (16) étant munie d'un deuxième élément de raccord (36, 38, 40) ou d'une pluralité de deuxièmes éléments de raccord au niveau de ladite quatrième extrémité de celle-ci destiné(s) à coopérer avec ledit premier élément de raccord de ladite première languette (14), et
un tampon (20, 64) d'absorption de liquide placé autour du centre de ladite première languette (14) ou bien alternativement de ladite deuxième languette (16) et destine à être placé au niveau de ladite zone de ladite extrémité sur ladite artère et/ou veine ouverte, et étant supporté par rapport à ladite première languette ou alternativement de ladite deuxième languette par un élément compressible ou flexible (66 ; 68) comprenant ou coopérant avec un élément indicateur (68 ; 64 ; 69) destiné à indiquer l'application d'une pression spécifique sur ladite zone de ladite extrémité au niveau de ladite artère et/ou veine ouverte par ledit tampon, et lesdites première et deuxième languettes (14, 16) étant positionnables encerclant circonférentiellement ladite extrémité (53, 54) afin d'appliquer ladite pression spécifique sur ladite zone au niveau de ladite artère et/ou veine ouverte par le réglage dudit verrouillage entre ladite première languette et ladite deuxième languette grâce auxdits premier et deuxième éléments de raccord.

2. Dispositif selon la revendication 1, ledit premier matériau de ladite première languette étant identique audit deuxième matériau de ladite deuxième languette et étant une matière plastique, et ledit joint (18) entre ladite première et ladite deuxième languettes étant constitué d'une charnière intégrée ou d'une charnière à lame en dit matériau (12) de languette.

3. Dispositif selon l'une quelconque des revendications 1 ou 2, ledit premier élément de raccord (32, 34, 39) étant constitué d'une broche découpée dans ladite première languette (14) et chacun de ladite pluralité de deuxièmes éléments de verrouillage (36, 38, 40) étant constitué d'un évidement ou d'un trou de ladite extension de ladite deuxième languette.

4. Dispositif selon l'une quelconque des revendications 1 à 3, ledit élément compressible ou flexible étant constitué d'un ou plusieurs éléments de tampon supplémentaires (66, 68) supportant ledit tampon (64) absorbant le liquide.

5. Dispositif selon la revendication 4, lesdits éléments de tampon supplémentaires (66, 68) comprenant un tampon incompressible (68) et un tampon compressible (66) et ledit tampon compressible (66) fournissant ladite indication par une réduction de l'épaisseur dudit tampon compressible selon un taux spécifique, tel que par exemple 50 % de l'épaisseur dudit tampon compressible à l'état non comprimé, ou alternativement par compression dudit tampon compressible jusqu'à une épaisseur correspondant à l'épaisseur dudit tampon incompressible (68) ou à l'épaisseur dudit tampon (64) absorbant le liquide.

6. Dispositif selon l'une quelconque des revendications 1 à 3, ledit élément compression ou flexible étant constitué d'un élément de ressort (70) fournissant ladite indication par le positionnement d'un indicateur (72) dudit élément de ressort par rapport à un repère préréglé (74).

7. Dispositif selon l'une quelconque des revendications 1 à 6, ladite deuxième languette étant constituée d'une bande de matière plastique flexible ou incurvable en matériau tissé ou intissé.

8. Dispositif selon l'une quelconque des revendications 1 à 7, ladite première languette étant prémoulée selon une configuration incurvée spécifique.

9. Dispositif selon l'une quelconque des revendications 1 à 8, ledit tampon (64) absorbant le liquide étant recouvert d'une feuille (60) perforée à coefficient de frottement élevé.
